# EUROPEAN PATENT APPLICATION

(11) **EP 0 823 256 A1**
(43) Date of publication of application: **11.02.1998**
(21) Application number: 96910201.1
(22) Date of filing: 19.04.1996
(51) Int. Cl.: A61K 38/18, C07K 14/475

(54) **PREVENTIVE AND/OR REMEDY FOR ISCHEMIC DISEASES**

(30) Priority: 21.04.1995 JP 96994/95
(71) Applicant: MITSUBISHI CHEMICAL CORPORATION, Chiyoda-ku, Tokyo 100 (JP)
(72) Inventor: GEMBA, Munekazu, Yawata-shi, Kyoto 614 (JP); YONEHANA, Tsutomu, Takatsuki-shi, Osaka 569-11 (JP)
(74) Representative: Kolb, Helga, Dr. Dipl.-Chem.
(86) International application number: JP9601065
(87) International publication number: WO9632960

(57) **Abstract**

Disclosed is an agent for preventing and/or treating ischemic diseases, which comprises a hepatocyte-growth factor (e.g., a proteinic factor having such physicochemical properties that 1) an estimated molecular weight by SDS-PAGE is about 76 to 92 Kdalton; 2) it has an activity of proliferating hepatocytes; 3) the above activity is lost by heat treatment at 80 °C for 10 minutes; 4) the above activity is lost by digestion treatment using trypsin or digestion treatment using chymotrypsin; 5) it has strong affinity for heparin, and the like) as an active ingredient.

HGF which an active ingredient has an action of suppressing a cell disorder in an ischemic disorder model so that it is effective as an agent for preventing and/or treating ischemic diseases.

## Description

### Technical field

This invention relates to an agent for preventing/treating ischemic diseases, more specifically to an agent for preventing and/or treating ischemic diseases, which contains a hepatocyte-growth factor (hereinafter sometimes abbreviated to "HGF") as an active ingredient.

### Background art

Acute renal failure refers to a state that a renal function is abruptly lowered and blood urea nitrogen (BUN) and serum creatinine are remarkably elevated. It has been known that this acute renal failure is frequently caused by ischemia. It is considered that in treatment of such acute renal failure, recovery of a renal function thereafter is determined by removing a cause to recover from renal ischemia and preventing tissue disorders accompanied with reperfusion after ischemia. Also, in treatments of ischemic diseases of other than kidney, such as ischemic heart diseases, ischemic cerebrovascular disorders and the like, importance of prevention of a reperfusion disorder as well as restarting of a bloodstream has been pointed out. Also, as a means for recovering a stopped or lowered organic function, organ transplantation has been carried out. At the time of transplantation, it is an important task how a transplanted organ is protected from ischemic disorders.

On the other hand, HGF has been found in the plasma of patients with fulminant hepatic failure as a human-derived proteinic factor which can promote the growth of hepatocytes in primary culture (Japanese Provisional Patent Publication No. 22526/1988, a human hepatocyte-growth factor is hereinafter sometimes abbreviated to "hHGF"). Thereafter, there have been reported an amino acid sequence of a hHGF protein and a gene (cDNA) coding the same (Japanese Provisional Patent Publication No. 72883/1991), and further a process for producing a recombinant hHGF protein (hereinafter sometimes abbreviated to "rhHGF") using this cDNA and a transformant (Japanese Provisional Patent Publication No. 285693/1991). It has been recognized that a rhHGF protein has an action of promoting the proliferation and function of hepatocytes *in vitro* (J. Clin. Invest., 87, 1853-1857 (1991)) and *in vivo* (Jpn. J. Pharmacol., 59 (suppl. 1), 137 (1992)). Further, target cells and tissues of HGF have widely been searched, and it has been found that various cells of epithelia (tubular epithelium, lung epithelium, biliary epithelium, stomach epithelium), fibroblasts and lymphocytes other than hepatocytes are reacted with HGF to change growth and kinetics thereof (Mitsubishi Kasei R & D Review, 7, 16-24 (1993)). Also, it has been clarified that a proto-oncogene c-met product functions as a receptor molecule on the above HGF target cells (Science, 251, 802-804 (1991)).

Incidentally, in Japanese Provisional Patent Publication No. 49246/1992, it has been reported that HGF is useful for renal diseases of mammals including human. An object of the same publication is "to provide a treatment agent which can accelerate the growth of renal cells, promote renal regeneration in chronic nephritis and ameliorate renal failure, a treatment agent which promotes compensatory hypertrophy of kidney, a prophylactic agent which prevents a renal disorder caused by a medicine, an agent which promotes the growth of cultured renal cells, and a diagnostic agent which diagnoses a renal function" (page 3, left lower column, line 1 to line 6 of the same publication). However, only a growth-promoting activity of HGF to proximal tubular cells is disclosed specifically. That is, in the same publication, a pharmaceutical effect based on a renal cell-growing action is merely estimated and shown, and the effect of HGF on ischemic renal diseases are not described at all.

The present inventors have searched and studied a direct effect of HGF on a renal cell disorder by HGF, and consequently found for the first time that HGF has an action of suppressing a cell disorder in an ischemic disorder model, to accomplish the present invention.

### Disclosure of the invention

That is, the gist of the present invention resides in an agent for preventing and/or treating ischemic diseases, which comprises HGF as an active ingredient.

### Best mode for practicing the invention

In the following, the present invention is explained in detail.

The agent for preventing and/or treating ischemic diseases of the present invention contains HGF as an active ingredient. As such HGF, there may be used natural HGF isolated/produced from body fluids and tissues derived from mammals such as human, rats and the like which have been known to contain HGF, or from cells which spontaneously produce HGF, and there may be also used a recombinant HGF obtained by introducing cDNA of said HGF into cells by the gene recombination method. As the active ingredient of the agent for preventing and/or treating ischemic diseases of the present invention, hHGF is preferably used.

A host in which recombinant HGF is produced is not particularly limited, and there may be mentioned, for example, *Escherichia coli*, *Bacillus subtilis*, yeast, mold, vegetable cells, insect cells, animal cells and the like. As a specific example, there may be mentioned placentae derived from the above mammals, hepatic tissues and blood of a patient with hepatopathy, fibroblast strains such as MRC-5 cells, IMR-9 cells and the like, or a host obtained from a produced strain or the like in which an expression vector containing cDNA which codes hHGF is introduced into a host such as CHO cells and the like according to the method described in Japanese Provisional Patent Publication No. 285693/1991. Also, in addition to the above natural or recombinant HGF itself, there may be used a precursor protein thereof and non-natural type HGF changed by substitution, deletion, insertion, modification or the like of partial amino acid of natural HGF within the range which does not impair an activity of growing hepatocytes. As such non-natural type HGF, there may be mentioned non-natural type HGFs described in Japanese Provisional Patent Publication No. 288899/1990, WO 90/10651, Japanese Provisional Patent Publications No. 130091/1991, No. 255096/1991 and No. 30000/1992, Nature, 342, 440-443 (1989) and the like.

In the present invention, HGF is preferably a proteinic factor having the following physicochemical properties described in Japanese Provisional Patent Publication No. 22526/1988 and U.S. Patent No. 5,004,805:
1) an estimated molecular weight by sodium dodecylsulfate-polyacrylamide gel electrophoresis (SDS-PAGE; under non-reduced conditions) is about 76,000 to 92,000 dalton,
2) it has an activity of proliferating hepatocytes,
3) the above activity is lost by heat treatment at 80 °C for 10 minutes,
4) the above activity is lost by digestion treatment using trypsin or digestion treatment using chymotrypsin, and
5) it has strong affinity for heparin.

Such HGF is preferably derived from human, and as particularly preferred examples, there may be mentioned HGF represented by an amino acid sequence shown by Sequence No. 1 in the sequence listing shown below, HGF represented by a sequence from the 30th glutamic acid to the 728th serine of the amino acid sequence of Sequence No. 1, and HGF represented by a sequence from the 32nd glutamine to the 728th serine of the amino acid sequence of Sequence No. 1, described in Japanese Provisional Patent Publication No. 72883/1991, Japanese Provisional Patent Publication No. 89499/1992 and European Patent Publication No. 0412557.

The agent for preventing and/or treating ischemic diseases of the present invention is used by compounding one or two or more of the above HGF alone or compounding it/them with a suitable diluent and other additives to have a formulation form (a preparation type). The preparation type is not particularly limited so long as it is a preparation type generally suitable for parenteral administration, and an ampoule for injection and a lyophilized powder agent (a vial) for injection are preferably used. Preparations into various kinds of preparation types are carried out by using a general means commonly used in this field of the art. As a formulation carrier to be used for preparing a formulation, there may be used a diluent, an additive and the like commonly used for preparing various kinds of preparation types. For example, the lyophilized powder agent for injection is prepared by, for example, dissolving an effective amount of the above HGF which has been purified, in a diluent such as distilled water, physiological saline, a glucose aqueous solution and the like, if necessary, adding an excipient such as carboxymethyl cellulose, sodium alginate and the like, a stabilizer such as polyethylene glycol, dextran sodium sulfate, amino acid, human serum albumin and the like, a preservative such as benzyl alcohol, benzalkonium chloride, phenol and the like, a pain-relieving agent such as glucose, calcium gluconate, procaine hydrochloride and the like, a pH adjustor such as hydrochloric acid, acetic acid, citric acid, sodium hydroxide and the like, and others to the solution, and lyophilizing the mixture according to a conventional method. Also, the ampoule for injection is prepared by, for example, dissolving an effective amount of the above HGF in a diluent such as distilled water, physiological saline, a Ringer's solution and the like, if necessary, adding a dissolving aid such as sodium salicylate, mannitol and the like, a buffer such as sodium citrate, glycerin and the like, an isotonicity-imparting agent such as glucose, invert sugar and the like, the above stabilizer, the above preservative, the above pain-relieving agent, the above pH adjustor and the like to the solution, and sterilizing the mixture by general heat sterilization, sterile filtration or the like. Depending on the kind of the active ingredient, it may inactivate by heat sterilization, sterile filtration or the like so that it is preferred to select a sterilization method suitably. Also, the amounts of the above stabilizer, preservative, pain-relieving agent, pH adjustor and the like to be formulated are suitably determined depending on various kinds of preparation types.

HGF which is the active ingredient of the agent for preventing and/or treating ischemic diseases of the present invention has an action of suppressing a cell disorder in an ischemic disorder model so that the agent for preventing and/or treating ischemic diseases of the present invention are effective for preventing and/or treating ischemic diseases such as reperfusion disorders of a bloodstream, ischemia reperfusion injuries at the time of kidney transplantation, acute renal failure, ischemic heart diseases, ischemic cerebrovascular disorders and the like. Also, it is effective as an agent for protecting a transplanted organ in an operation for transplantation of an organ such as kidney, liver, heart, blood vessel and the like, i.e. effective for preventing and/or treating ischemic diseases such as organopathy and the like at the time of organ transplantation.

Into the agent for preventing and/or treating ischemic diseases of the present invention, there may be formulated other medical effective component having the same pharmaceutical action as that of the present invention or other pharmaceutical action may be formulated.

Also, in the agent for preventing and/or treating ischemic diseases of the present invention, as described in Japanese Provisional Patent Publication No. 301824/1993 and European Patent Publication No. 0517182, by using HGF in combination with a sulfated polysaccharide such as heparin, dextran sulfate and the like or a derivative thereof, its activity can be reinforced and can be also stabilized.

A predetermined amount at one time or divided into plural doses of the agent for preventing and/or treating ischemic diseases of the present invention is administered to a patient who needs this agent perenterally, in general, by subcutaneous, intramuscular or intravenous injection, or administered continuously by instillation or the like. Such an amount to be administered is suitably adjusted depending on the age, sex distinction, disease condition, body weight and the like of a patient, but the agent of the present invention is administered in an amount in the range of 1 µg/kg to 10 mg/kg, more preferably 10 to 1000 µg/kg per day as an effective HGF amount in the case of an adult.

### Examples

In the following, the present invention is explained in more detail by referring to Examples, but the present invention is not limited to the following Examples unless it does not fall outside the gist thereof. As HGF, rhHGF prepared by using a BD-24 strain described in Japanese Provisional Patent Publication No. 285693/1991 was used.

### Example 1

A culture epithelial cell line LLC-PK₁ (obtained from American Type Culture Collection) derived from pig kidney, which was known to be strongly damaged by ischemic acute renal failure and had properties like those of a proximal uriniferous tubule, was cultured in a medium in which bovine fetal serum and N-2-hydroxyethylpiperazine ethanesulfonic acid (HEPES) were added to a Dulbecco modified Eagle's medium (DMEM) so that the concentrations thereof were 5 % and 10 mM, respectively, by using a CO₂ incubator until cells reached confluence.

After the medium was replaced with DMEM containing neither glucose nor serum, the concentration of oxygen was lowered to less than 2 % within 2 hours after initiation of the experiment by using Gas Pak Pouch™ (manufactured by BBL Co.). The cells after confluence were cultured for 6 hours under this low oxygen condition and then cultured for 1 hour under a general oxygen-containing condition (95 % air, 5 % CO₂) (re-oxygenation).

The disorder of the cells was judged by using an amount of lactate dehydrogenase (LDH) leaked into the medium as an index (Kidney and Free Radical, the second series, pp. 139 to 141, Tokyo Igakusha, December 1994).

50 or 100 ng/ml of HGF was added to the medium immediately before hypoxia or immediately before re-oxygenation. When HGF was not added, increase in the amount of LDH liberating into the medium was recognized by lowering of oxygen or re-oxygenation. An action exerted on the LDH liberation amount when HGF was added was measured under the above conditions. The measurement results are shown in Table 1. The results are shown as a relative amount when the liberated LDH amount when HGF was not added was defined as 100 %.

**Table 1**

| Addition amount of HGF | 50 ng/ml | 100 ng/ml |
|---|---|---|
| HGF added immediately before lowering of oxygen | 49.6 % | 39.0 % |
| HGF added immediately before re-oxygenation | 60.9 % | 57.0 % |

As shown above, the LDH liberation amount was remarkably suppressed by adding HGF to the medium immediately before lowering of oxygen or immediately before re-oxygenation.

As described above, in the cells which reached confluence, an effect exhibits in at least one hour after addition of HGF so that it is apparent that HGF has a cell disorder-suppressing action other than a cell growth-promoting action, on the LLC-PK₁ cells.

### Utilizability in industry

The prophylactic and/or treatment agent of the present invention has an action of suppressing a cell disorder in an ischemic disorder model so that it is effective as an agent for preventing and/or treating ischemic diseases.

Sequence listing

Sequence No.: 1
Length of sequence: 728
Type of sequence: amino acid
Strandness: single strand
Topology: linear
Kind of sequence: protein
Original source:
Name of organism: human

Sequence

Met Trp Val Thr Lys Leu Leu Pro Ala Leu Leu Leu Gln His Val Leu
1               5                   10                  15
Leu His Leu Leu Leu Leu Pro Ile Ala Ile Pro Tyr Ala Glu Gly Gln
            20                  25                  30
Arg Lys Arg Arg Asn Thr Ile His Glu Phe Lys Lys Ser Ala Lys Thr
        35                  40                  45
Thr Leu Ile Lys Ile Asp Pro Ala Leu Lys Ile Lys Thr Lys Lys Val
    50                  55                  60
Asn Thr Ala Asp Gln Cys Ala Asn Arg Cys Thr Arg Asn Lys Gly Leu
65                  70                  75                  80
Pro Phe Thr Cys Lys Ala Phe Val Phe Asp Lys Ala Arg Lys Gln Cys
                85                  90                  95
Leu Trp Phe Pro Phe Asn Ser Met Ser Ser Gly Val Lys Lys Glu Phe
            100                 105                 110
Gly His Glu Phe Asp Leu Tyr Glu Asn Lys Asp Tyr Ile Arg Asn Cys
        115                 120                 125
Ile Ile Gly Lys Gly Arg Ser Tyr Lys Gly Thr Val Ser Ile Thr Lys
    130                 135                 140
Ser Gly Ile Lys Cys Gln Pro Trp Ser Ser Met Ile Pro His Glu His
145                 150                 155                 160
Ser Phe Leu Pro Ser Ser Tyr Arg Gly Lys Asp Leu Gln Glu Asn Tyr
                165                 170                 175

Cys Arg Asn Pro Arg Gly Glu Glu Gly Gly Pro Trp Cys Phe Thr Ser
            180                 185                 190
Asn Pro Glu Val Arg Tyr Glu Val Cys Asp Ile Pro Gln Cys Ser Glu
        195                 200                 205
Val Glu Cys Met Thr Cys Asn Gly Glu Ser Tyr Arg Gly Leu Met Asp
    210                 215                 220
His Thr Glu Ser Gly Lys Ile Cys Gln Arg Trp Asp His Gln Thr Pro
225                 230                 235                 240
His Arg His Lys Phe Leu Pro Glu Arg Tyr Pro Asp Lys Gly Phe Asp
                245                 250                 255
Asp Asn Tyr Cys Arg Asn Pro Asp Gly Gln Pro Arg Pro Trp Cys Tyr
            260                 265                 270
Thr Leu Asp Pro His Thr Arg Trp Glu Tyr Cys Ala Ile Lys Thr Cys
        275                 280                 285
Ala Asp Asn Thr Met Asn Asp Thr Asp Val Pro Leu Glu Thr Thr Glu
    290                 295                 300
Cys Ile Gln Gly Gln Gly Glu Gly Tyr Arg Gly Thr Val Asn Thr Ile
305                 310                 315                 320
Trp Asn Gly Ile Pro Cys Gln Arg Trp Asp Ser Gln Tyr Pro His Glu
                325                 330                 335
His Asp Met Thr Pro Glu Asn Phe Lys Cys Lys Asp Leu Arg Glu Asn
            340                 345                 350
Tyr Cys Arg Asn Pro Asp Gly Ser Glu Ser Pro Trp Cys Phe Thr Thr
        355                 360                 365
Asp Pro Asn Ile Arg Val Gly Tyr Cys Ser Gln Ile Pro Asn Cys Asp
    370                 375                 380
Met Ser His Gly Gln Asp Cys Tyr Arg Gly Asn Gly Lys Asn Tyr Met
385                 390                 395                 400
Gly Asn Leu Ser Gln Thr Arg Ser Gly Leu Thr Cys Ser Met Trp Asp
                405                 410                 415
Lys Asn Met Glu Asp Leu His Arg His Ile Phe Trp Glu Pro Asp Ala
            420                 425                 430
Ser Lys Leu Asn Glu Asn Tyr Cys Arg Asn Pro Asp Asp Asp Ala His
        435                 440                 445
Gly Pro Trp Cys Tyr Thr Gly Asn Pro Leu Ile Pro Trp Asp Tyr Cys
    450                 455                 460

Pro Ile Ser Arg Cys Glu Gly Asp Thr Thr Pro Thr Ile Val Asn Leu
465                 470                 475                 480
Asp His Pro Val Ile Ser Cys Ala Lys Thr Lys Gln Leu Arg Val Val
                485                 490                 495
Asn Gly Ile Pro Thr Arg Thr Asn Ile Gly Trp Met Val Ser Leu Arg
            500                 505                 510
Tyr Arg Asn Lys His Ile Cys Gly Gly Ser Leu Ile Lys Glu Ser Trp
        515                 520                 525
Val Leu Thr Ala Arg Gln Cys Phe Pro Ser Arg Asp Leu Lys Asp Tyr
    530                 535                 540
Glu Ala Trp Leu Gly Ile His Asp Val His Gly Arg Gly Asp Glu Lys
545                 550                 555                 560
Cys Lys Gln Val Leu Asn Val Ser Gln Leu Val Tyr Gly Pro Glu Gly
                565                 570                 575
Ser Asp Leu Val Leu Met Lys Leu Ala Arg Pro Ala Val Leu Asp Asp
            580                 585                 590
Phe Val Ser Thr Ile Asp Leu Pro Asn Tyr Gly Cys Thr Ile Pro Glu
        595                 600                 605
Lys Thr Ser Cys Ser Val Tyr Gly Trp Gly Tyr Thr Gly Leu Ile Asn
    610                 615                 620
Tyr Asp Gly Leu Leu Arg Val Ala His Leu Tyr Ile Met Gly Asn Glu
625                 630                 635                 640
Lys Cys Ser Gln His His Arg Gly Lys Val Thr Leu Asn Glu Ser Glu
                645                 650                 655
Ile Cys Ala Gly Ala Glu Lys Ile Gly Ser Gly Pro Cys Glu Gly Asp
            660                 665                 670
Tyr Gly Gly Pro Leu Val Cys Glu Gln His Lys Met Arg Met Val Leu
        675                 680                 685
Gly Val Ile Val Pro Gly Arg Gly Cys Ala Ile Pro Asn Arg Pro Gly
    690                 695                 700
Ile Phe Val Arg Val Ala Tyr Tyr Ala Lys Trp Ile His Lys Ile Ile
705                 710                 715                 720
Leu Thr Tyr Lys Val Pro Gln Ser
                725

## Claims

1. An agent for preventing and/or treating ischemic diseases, which comprises a hepatocyte-growth factor as an active ingredient.

2. The agent for preventing and/or treating ischemic diseases according to Claim 1, wherein the ischemic disease is a reperfusion disorder of a bloodstream.

3. The agent for preventing and/or treating ischemic diseases according to Claim 1, wherein the ischemic disease is an ischemia reperfusion injury at the time of kidney transplantation.

4. The agent for preventing and/or treating ischemic diseases according to Claim 1, wherein the ischemic disease is acute renal failure.

5. The agent for preventing and/or treating ischemic diseases according to Claim 1, wherein the ischemic disease is organopathy at the time of organ transplantation.

6. The agent for preventing and/or treating ischemic diseases according to Claim 1, wherein the hepatocyte-growth factor is a proteinic factor having the following physicochemical properties:
1) an estimated molecular weight by sodium dodecylsulfate-polyacrylamide gel electrophoresis (SDS-PAGE; under non-reduced conditions) is about 76,000 to 92,000 dalton,
2) it has an activity of proliferating hepatocytes,
3) the above activity is lost by heat treatment at 80 °C for 10 minutes,
4) the above activity is lost by digestion treatment using trypsin or digestion treatment using chymotrypsin, and
5) it has strong affinity for heparin.

7. The agent for preventing and/or treating ischemic diseases according to Claim 1, wherein the hepatocyte-growth factor is a hepatocyte-growth factor derived from human.

8. The agent for preventing and/or treating ischemic diseases according to Claim 1, wherein the hepatocyte-growth factor is a recombinant hepatocyte-growth factor using cDNA.

9. The agent for preventing and/or treating ischemic diseases according to Claim 8, wherein the recombinant hepatocyte-growth factor is a hepatocyte-growth factor represented by an amino acid sequence shown by Sequence No. 1.

10. The agent for preventing and/or treating ischemic diseases according to Claim 8, wherein the recombinant hepatocyte-growth factor is a hepatocyte-growth factor represented by a sequence from the 30th glutamic acid to the 728th serine of an amino acid sequence shown by Sequence No. 1.

11. The agent for preventing and/or treating ischemic diseases according to Claim 8, wherein the recombinant hepatocyte-growth factor is a hepatocyte-growth factor represented by a sequence from the 32nd glutamine to the 728th serine of an amino acid sequence shown by Sequence No. 1.

12. The agent for preventing and/or treating ischemic diseases according to Claim 1, wherein the hepatocyte-growth factor is administered in an amount in the range of 1 µg/kg to 10 mg/kg per day in the case of an adult.

13. The agent for preventing and/or treating ischemic diseases according to Claim 1, wherein the hepatocyte-growth factor is administered in an amount in the range of 10 to 1000 µg/kg per day in the case of an adult.
